# EUROPEAN PATENT SPECIFICATION

(11) **EP 4 007 621 B1**
(45) Date of publication and mention of the grant of the patent: **02.09.2026**
(21) Application number: 20743346.7
(22) Date of filing: 13.07.2020
(51) Int. Cl.: A61M 5/142, A61M 25/01, A61M 25/02, A61L 29/16

(54) **KIT WITH AN INSERTION DEVICE AND AN ANTIMICROBIAL CONTACT DRESSING**
SET MIT EINFÜHRVORRICHTUNG UND ANTIMIKROBIELLEM KONTAKTVERBAND
KIT COMPRENANT UN DISPOSITIF D'INSERTION ET UN PANSEMENT DE CONTACT ANTIMICROBIEN

(30) Priority: 01.08.2019 US 201962881650 P
(43) Date of publication of application: 08.06.2022
(73) Proprietor: Solventum Intellectual Properties Company, Eagan, MN 55121 (US)
(72) Inventor: HOMMES, Joseph M., Saint Paul, Minnesota 55133-3427 (US)
(74) Representative: Vossius & Partner Patentanwälte Rechtsanwälte mbB
(86) International application number: PCT/IB2020/056565
(87) International publication number: WO 2021/019339

(56) References cited:
- WO-A1-01/68179
- US-A1- 2015 297 867
- US-A1- 2016 206 855
- US-B1- 9 566 417

## Description

### Technical Field

The present disclosure relates to a kit with an insertion device and an antimicrobial contact dressing, and a process of providing an antimicrobial agent around an insertion device.

### Background

Various tubes, pins, or other percutaneous devices pass through skin for a variety of functions, such as fluid delivery or medical device support. There are several types of percutaneous devices used to administer medication, such as, for example, a diabetic pump or a Huber needle, which might also include an implanted port. Some of these devices may be connected for an extended period of time causing irritation or discomfort on the skin and increasing the risks of infection at the injection site. For instance, US 9,566,417 is directed to an integrated antimicrobial dressing designed for catheter care, and 2 US 2016/206855 describes securement systems for medical articles, such as catheters.

### Summary

The disclosed kit comprises an access needle hub comprising a housing and an insertion needle; and an antimicrobial device, comprising: a contact dressing comprising: a contact dressing first surface comprising an adhesive for securing the antimicrobial device to a substrate and an antimicrobial agent; and a contact dressing second surface, opposite the contact dressing first surface; wherein the housing is adjacent the contact dressing second surface and the insertion needle is inserted through the contact dressing; wherein the adhesive and the antimicrobial agent are located on the contact dressing first surface adjacent the inserted insertion needle; and wherein the insertion needle is configured to puncture through the contact dressing and into the substrate.

### Brief Description of Drawings

FIG. 1 is a side sectional view of one embodiment of an antimicrobial device;
FIG. 2 is a plan view of one embodiment of a first contact dressing surface;
FIG. 3 is a plan view of another embodiment of a first contact dressing surface;

While the above-identified drawings and figures set forth embodiments of the invention, other embodiments are also contemplated, as noted in the discussion. In all cases, this disclosure presents the invention by way of representation and not limitation.

The figures may not be drawn to scale.

### Detailed Description

A conventional device to providing antimicrobial protection to an insertion needle is a disk-shaped sponge loaded with an antimicrobial agent, commercially available as Biopatch^{™} protective disk available from Ethicon. To use the disk-shaped sponge, the tube or insertion needled is first threaded through the opening of the disk, and then the insertion needle is inserted into the substrate, typically skin. This requires two hands working together to thread the insertion needle through the disk. Also, the disk-shaped device is not transparent. Therefore, the disk-shaped device must be removed to view the area around the insertion needle for signs of infection. Another device that provides antimicrobial protection to an insertion needle is 3M Tegaderm CHG dressing, which includes a gel pad loaded with chlorhexidine gluconate. For this device, the needle is inserted and the gel pad is placed over the inserted needle.

The disclosed antimicrobial device 100 has an antimicrobial contact dressing 110 that securely attaches to an underlying substrate 140. The antimicrobial contact dressing 110 has a surface with an adhesive 114 and an antimicrobial agent 115. An insertion needle 134 can puncture though the antimicrobial contact dressing 110 for easy application and antimicrobial protection of the insertion needle 134. The adhesive 114 and antimicrobial agent 115 are adjacent to the inserted needle 134. The adhesive 114 maintains the contact dressing 110 securely in place to the underlying substrate 140 follow insertion of the insertion needle 134, and the antimicrobial agent 115 also being adjacent to the insertion needle 134 maintains antimicrobial efficacy to the area around the insertion needle 115.

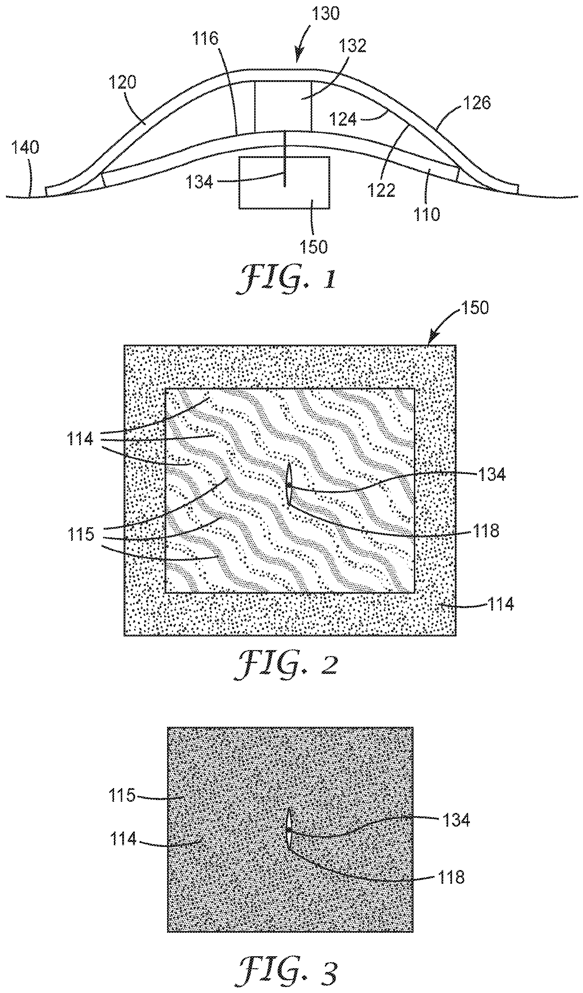

FIG. 1 is a side sectional view of one embodiment of an antimicrobial device 100. The antimicrobial device has a contact dressing 110 that secures to an underlying substrate 140. The contact dressing 110 has a contact dressing first surface 112 with an adhesive 114 and antimicrobial agent 115. The adhesive 114 on the contact dressing first surface 112 secures the contact dressing 110 to the underlying substrate 140. Opposite from the contact dressing first surface 112, is the contact dressing second surface 116. Typically, the contact dressing 110 is a thin, flexible, conformable material with one or more layers, such as a film, paper, foil, woven, knitted, or nonwoven.

An access needle hub 130 includes a housing 132 and insertion needle 134. In use, the housing 132 is adjacent to the contact dressing second surface 116. The insertion needle 134 punctures through the contact dressing 110 and into the substrate 140. Optionally, underlying the substrate 140 may be an access port 150 for receiving the insertion needle 134 and aligning with the housing 132. The access needle hub 130 can be used to deliver fluid through the insertion needle 134 and into the substrate 140 and/or can be used to remove fluid from the underlying substrate 140. The fluid flow through the access needle hub 130 could be a one-time delivery of fluid or could be continuously delivered.

To use the antimicrobial device 100, the contact dressing first surface 112 is applied to a substrate 140. After the contact dressing 110 is applied, the access needle hub 130 is placed over the contact dressing second surface 116. The insertion needle 134 passes through the contact dressing 110.

**In** one embodiment, the insertion needle 134 is protruding from the housing 130 and punctures though the contact dressing 110 as the access needle hub 130 comes into contact with the contact dressing second surface 110. The insertion needle 134 can be linear or slightly curved at the end. A linear insertion needle 134, often referred to as a non-coring needle, will tend to create a punch hole in the contact dressing 110. When the insertion needle 134 curved at the end, the insertion needle 134 will slice through the contact dressing 110 and will tend to create a linear slit 118 in the contact dressing 110.

**In** one embodiment, the insertion needle 134 is retracted within the housing 132 and as the access needle hub 130 comes into contact with the contact dressing second surface 116 the insertion needle 134 is actuated to release from within the housing 132. To release the insertion needle 134 from the housing 132, the insertion needle 134 can linearly projected out of the housing 132 and into the substrate 140. The insertion needle 134 can be either linear or slightly curved at the end. Linearly projecting the linear insertion needle 134 from the housing 140 will tend to create a punch hole in the contact dressing 110. Linearly projecting a slightly curved insertion needle 134 will tend to slice through the contact dressing 110 to create a slit 118. Alternatively, to release the insertion needle 134 from the housing, the insertion needle 134 will rotationally project out of the housing 132, pass through the contact dressing 110, and enter into the substrate 140. The rotational projection of the insertion needle 134 from the housing 132 will tend to create a linear slit 118 in the contact dressing 110.

The antimicrobial device 100 with the contact dressing 110 and an overlying access needle hub 130 that punctures through the contact dressing 118 to form a slit 118 can be advantageous over insertion needles 134 that puncture straight linearly. When passing a linear insertion needle 134 linearly through a material, it is possible that a small amount of the material could become lodged into the insertion needle 134 and then exit in the substrate 140. Non coring insertion needles 134 minimize this problem. Non coring insertion needles 134 will tend to create a slit 118 though the contact dressing 110.

The adhesive 114 and antimicrobial agent 115 are included at least in the area surrounding the insertion needle 134 and with both the adhesive and the antimicrobial agent 115 in contact with the substrate 140. The adhesive 114 and antimicrobial agent 115 may be applied to the contact dressing first surface 112 separately and adjacent to one another or the antimicrobial agent 115 is loaded into the adhesive 114.

Because the adhesive 114 is present at least in the area surrounding the insertion needle 134, the adhesive 114 secures the contact dressing 110 to the substrate 140. Then during and after the insertion needle 134 punctures through the contact dressing 110, the adhesive 114 maintains the contact dressing 110 in contact with the substrate 140. Therefore, the contact dressing 110 does not become loose or able to be dislodges and inadvertently enter into the substrate 140. Especially in embodiments of a non coring insertion needle 134 where a slit 118 is formed through the contact dressing 110, the contact dressing 110 will tend to widen at the slit 118 and the underlying adhesive 114 on the contact dressing 110 will maintain secure connection of the contact dressing first surface 112 at the slit 118.

Also, because the antimicrobial agent 115 is present at least in the area surrounding the insertion needle 134, the antimicrobial agent 115 is present at the interface of the insertion needle 134 and substrate 140 to aid in minimizing contamination and reducing the risk of infection.

As shown in FIG. 1, an optional cover dressing 120 can be used to aid in securing the access needle hub 130. The cover dressing 120 has a cover dressing first surface 122 and an opposite cover dressing second surface 126. The cover dressing first surface 122 has a cover dressing adhesive 124 to allow the cover dressing to secure to the underlying access needle hub 130, the contact dressing second surface 116, and/or to the substrate 140.

FIG. 2 is a plan view of one embodiment of a contact dressing first surface 112 showing the insertion needle 134, which has formed a slit 118 though the contact dressing first surface 112. In this embodiment, the adhesive 114 and antimicrobial agent 115 are located adjacent to the slit 118 and adjacent to the insertion needle 134. In this embodiment, the adhesive 114 is distinct from the antimicrobial agent 115. As shown in FIG. 2, each of the adhesive 114 and the antimicrobial agent 115 is applied in a pattern and separate from one another. As shown in this embodiment, the perimeter region comprises the adhesive 114, and does not include the antimicrobial agent 115.

FIG. 3 is a plan view of one embodiment of a contact dressing first surface 112 showing the insertion needle 134, which has formed a slit 118 though the contact dressing first surface 112. In this embodiment, the adhesive 114 and antimicrobial agent 115 are located adjacent to the slit 118 and adjacent to the insertion needle 134. In this embodiment, the antimicrobial agent 115 is dispersed throughout the adhesive 114. As shown in FIG. 3, the antimicrobial-containing adhesive 114 is uniformly applied over the contact dressing first surface 112. In other embodiments, the antimicrobial-containing adhesive 114 may be applied discontinuously, such as in a pattern on the contact dressing first surface 112.

Typically, to provide the antimicrobial device 100 may be provided to a health care provider in a kit with a access needle hub 130. Optionally, a cover dressing and tape can be included in the kit. Optionally, skin antiseptic materials can be included in the kit to clean the skin prior to application of the contact dressing 110. Optionally, gloves or masks can be provided in the kits for the health care provider to wear while applying the contact dressing and inserting the access needle hub. Typically, components in the kit are sterile or the entire kit is sterilized.

### Contact dressing/Cover dressing

The contact dressing 110 and cover dressing 120 are typically thin film materials, coated with the adhesive. Typically, the thin film material provides resistance against incoming water and contaminants and has a high moisture vapor permeability to allow moisture vapor from the underlying skin to exit. One example of a suitable material is a high moisture vapor permeable film such as described in US Pat 3,645,835 and 4,595,001. Issued U.S. Pat. Nos. 3,645,835 and 4,595,001 describe methods of making such films and methods for testing their permeability. Typically, the film/adhesive composite should transmit moisture vapor at a rate equal to or greater than human skin. Typically, the adhesive coated film transmits moisture vapor at a rate of at least 300 g/m² /24 hrs/37°C/100-10% RH, more preferably at least 700 g/m² /24 hrs/37°C/100-10% RH, and most preferably at least 2000 g/m² /24 hrs/37°C/100-10% RH using the inverted cup method as described in U.S. Pat. No. 4,595,001.

The material for the dressing is preferably conformable to anatomical surfaces. As such, when applied to an anatomical surface, it conforms to the surface even when the surface is moved and can stretch and retract. One embodiment of a film is elastomeric polyurethane, polyester, or polyether block amide films. These films combine the desirable properties of resiliency, high moisture vapor permeability, and transparency. Example of material for the thin film dressing is in 3M Tegaderm IV Dressings available from 3M Company. US Patent application 62/783,368, filed December 21, 2018 describes a medical article with backing that may be suitable as the contact dressing.

Additional materials can be included with the thin film to give the dressing strength, rigidity. For example, woven, knitted, nonwoven, or polymeric material can be included. US Patent Application Publication US 2016-0015570. Examples of multilayer constructions for a dressing is in 3M Tegaderm IV Advanced Dressings, available from 3M Company.

Typically, thin film dressing includes a frame. The frame is made of a substrate that is less resilient than the film and may be removable from the film. Examples of materials and frames include those in 3M Tegaderm IV Dressings and 3M Tegaderm IV Advanced Dressings, both available from 3M Company.

### Antimicrobial agent

The contact dressing first surface 112 contains an antimicrobial agent 115 to deliver antimicrobial activity to the skin in and around the injection site, reducing the likelihood of an infection. There are numerous biologically active materials, which include antimicrobial agents. The antimicrobial agent 115 can be applied directly to the contact dressing first surface 112 adjacent to the adhesive 114 or may be incorporated into the adhesive 114. In some embodiments, the antimicrobial agent 115 may be applied in a pattern. Also, the antimicrobial agent 115 may be applied to the entire contact dressing first surface.

Examples of antimicrobial agents include parachlorometaxylenol; triclosan; chlorhexidine and its salts such as chlorhexidine gluconate, poly hexamethylene biguanide and its salts such as poly hexamethylene biguanidine chloride, iodine, idodophors; fatty acid monoesters; poly-n-vinyl pyrrolidone-iodophors; silver oxide, silver and its salts, peroxides (e.g. hydrogen peroxide), antibiotics (e.g. neomycin, bacitracin, and polymixin B).

The following active ingredients could also be used to suppress the regrowth or possibly treat an infection of microorganisms in the present invention: 2,2-thiobis(4-chlorophenol); 4,4-isopropylidenediphenol; 5-amino-6-chloro-o-cresol; acetaminosalol; alcloxa; aldioxa; aluminum acetate; aluminum benzoate; aluminum diacetate; aluminum formate; aluminum phenolsulfonate; ammonium iodide; ammonium phenolsulfonate; benzisothiazolinone; benzotriazole; benzoxiquine; benzylparaben; berberine chloride; boric acid; cetethyl morpholinium ethosulfate; cetethyldimonium bromide; cetrimonium tosylate; cetylpyridinium chloride; chloramine-t; chlorothymol; cloflucarban; cocotrimonium chloride; colloidal sulfur; copper usnate; dedm hydantoin; dedm hydantoin dilaurate; dequalinium acetate; dequalinium chloride; dibromopropamidine diisethionate; dichloro-m-xylenol; dichlorophene; dichlorophenyl imidazoldioxolan; diiodomethyltolylsulfone; dimethyl hydroxymethyl pyrazole; dimethylaminostyryl heptyl methyl thiazolium iodide; dodecylbenzyltrimonium chloride; domiphen bromide; ferulic acid; fluorosalan; glyoxal; hydroxymethyl dioxoazabicyclooctane; hydroxypropyl bistrimonium diiodide; ichthammol; isodecylparaben; isopropyl sorbate; lapyrium chloride; laurtrimonium trichlorophenoxide; lauryl isoquinolinium bromide; lauryl isoquinolinium saccharinate; laurylpyridinium chloride; m-cresol; mandelic acid; MDM hydantoin; MEAa-iodine; melaleuca alternifolia; methylbenzethonium chloride; mixed cresols; nonoxynol-12 iodine; nonoxynol-9 iodine; o-cresol; oxyquinoline benzoate; oxyquinoline sulfate; p-chlorophenol; p-cresol; PEG-15 dedm hydantoin; PEG-15 dedm hydantoin stearate; PEG-5 dedm hydantoin; PEG-5 dedm hydantoin oleate; phenol; phenoxyethylparaben; phenyl salicylate; polymethoxy bicyclic oxazolidine; potassium iodide; potassium lactate; potassium phenoxide; potassium troclosene; quaternium-14; quatemium-24; quatemium-8; ricinoleamidopropyltrimonium methosulfate; sodium iodide; sodium p-chloro-m-cresol; sodium phenolsulfonate; sodium phenoxide; sodium usnate; steapyrium chloride; strontium peroxide; tea-sorbate; tetrabutyl ammonium bromide; thiabendazole; triacetin; undecylenamide dea; undecylenamide mea; undecylenamidopropyltrimonium methosulfate; undecyleneth-6; undecylenoyl peg-5 paraben; usnic acid; zinc acetate; zinc borate; zinc phenolsulfonate; zinc sulfate; zinc undecylenate; and combinations of the foregoing.

The following actives could also be of use to also reduce regrowth of microorganisms on skin: 2-bromo-2-nitropropane-1,3-diol; 4-hydroxybenzoic acid; 5-bromo-5-nitro-1,3-dioxane; 7-ethylbicyclooxazolidine; ammonium benzoate; ammonium bisulfite; ammonium propionate; ammonium sulfite; behentrimonium chloride; benzalkonium bromide; benzalkonium chloride; benzalkonium saccharinate; benzethonium chloride; benzoic acid; benzyl alcohol; benzylhemiformal; bromochlorophene; butyl benzoate; butylparaben; calcium benzoate; calcium paraben; calcium propionate; calcium salicylate; calcium sorbate; calcium undecylenate; cetalkonium chloride; cetearalkonium bromide; cetrimonium bromide; cetrimonium chloride; chloroacetamide; chlorobutanol; chlorophene; chloroxylenol; chlorphenesin; climbazole; dehydroacetic acid; diazolidinyl urea; dibromohexamidine isethionate; dichlorobenzyl alcohol; dimethyl oxazolidine; DMDM hydantoin; ethyl benzoate; ethylparaben; formaldehyde; formic acid; glutaral; hexamidine; hexamidine diisethionate; hexamidine paraben; hexetidine; hydrogenated tallowtrimonium chloride; imidazolidinyl urea; iodopropynyl butylcarbamate; isobutyl benzoate; isobutylparaben; isopropyl benzoate; isopropyl cresols; isopropylparaben; lauralkonium bromide; lauralkonium chloride; laurtrimonium bromide; laurtrimonium chloride; magnesium benzoate; magnesium propionate; magnesium salicylate; MEA o-phenylphenate; MEA-benzoate; MEA-salicylate; MEA-undecylenate; methenamine; methyl benzoate; methylchloroisothiazolinone; methyldibromo glutaronitrile; methylisothiazolinone; methylparaben; myristalkonium chloride; myristalkonium saccharinate; myrtrimonium bromide; o-cymen-5-ol; o-phenylphenol; olealkonium chloride; p-chloro-m-cresol; phenoxyethanol; phenoxyisopropanol; phenyl benzoate; phenyl mercuric acetate; phenyl mercuric benzoate; phenyl mercuric borate; phenyl mercuric bromide; phenyl mercuric chloride; phenylparaben; piroctone olamine; polyaminopropyl biguanide; potassium benzoate; potassium butylparaben; potassium ethylparaben; potassium metabisulfite; potassium methylparaben; potassium o-phenylphenate; potassium paraben; potassium propionate; potassium propylparaben; potassium salicylate; potassium sorbate; potassium sulfite; propionic acid; propyl benzoate; propylparaben; quatemium-15; salicylic acid; sodium benzoate; sodium bisulfite; sodium butylparaben; sodium dehydroacetate; sodium ethylparaben; sodium formate; sodium hydroxymethylglycinate; sodium iodate; sodium metabisulfite; sodium methylparaben; sodium o-phenylphenate; sodium paraben; sodium propionate; sodium propylparaben; sodium salicylate; sodium sorbate; sodium sulfite; sodium undecylenate; sorbic acid; soytrimonium chloride; stearalkonium chloride; steartrimonium chloride; tallowalkonium chloride; tallowtrimonium chloride; thimerosal; triclocarban; triclosan; undecylenic acid; zinc pyrithione; and combinations of the foregoing.

### Adhesive

The adhesive used on the contact dressing and/or cover dressing is typically a pressure sensitive adhesive, such as an acrylate-based adhesive. The pressure sensitive adhesives described above typically transmit moisture vapor at a rate greater to or equal to that of human skin.

One example of a pressure sensitive acrylate adhesive that can be applied to skin is described in U.S. Patent No. RE 24,906. In one embodiment, a 97:3 iso-octyl acrylate:acrylamide copolymer adhesive can be used or a 70:15:15 isooctyl acrylate: ethyleneoxide acrylate:acrylic acid terpolymer, as described in U.S. Pat. No. 4,737,410 (Example 31). Other useful adhesives are described in U.S. Pat. Nos. 3,389,827, 4,112,213, 4,310,509, and 4,323,557. Silicone adhesive can also be used. Generally, silicone adhesives can provide suitable adhesion to skin while gently removing from skin. Suitable silicone adhesives are disclosed in PCT Publications WO2010/056541 and WO2010/056543.

US Patent Application Publications 2018/0280591 and 2015/0238444 disclose antimicrobial agents dispersed throughout an adhesive composition. For example, chlorohexidine gluconate can be included within the pressure-sensitive acrylate adhesive to provide continuous antimicrobial activity.

The adhesive 114 can be applied directly to the contact dressing first surface 112 adjacent to the antimicrobial agent 115 or the antimicrobial agent 115 may be incorporated into the adhesive 114. In some embodiments, the adhesive may be applied in a pattern, as described in U.S. Pat. No. 4,595,001. Also, it is understood that the entire surface may include adhesive or the adhesive may be at select portions.

Optionally a release liner maybe used to cover the adhesive. During use, the release liner is removed, exposing the adhesive. Release liners can be made of paper or films, such as kraft papers, polyethylene, polypropylene, polyester or composites of any of these materials. The release liners are typically coated with release agents such as fluorochemicals or silicones.

Although specific embodiments have been shown and described herein, it is understood that these embodiments are merely illustrative of the many possible specific arrangements that can be devised in application of the principles of the invention. Numerous and varied other arrangements can be devised in accordance with these principles by those of skill in the art without departing from the scope of the invention. The scope of the present invention should be limited only by the structures described by the language of the claims.

## Claims

1. A kit, comprising
an access needle hub (130) comprising a housing (132) and an insertion needle (134); and
an antimicrobial device (100), comprising:
a contact dressing (110) comprising:
a contact dressing first surface (112) comprising an adhesive (114) for securing the antimicrobial device to a substrate (140) and an antimicrobial agent (115); and
a contact dressing second surface (116), opposite the contact dressing first surface;
wherein the housing (132) is adjacent the contact dressing second surface (116) and the insertion needle (134) is inserted through the contact dressing (110);
wherein the adhesive (114) and the antimicrobial agent (115) are located on the contact dressing first surface adjacent the inserted insertion needle (134); and
**characterised in that** the insertion needle (134) is configured to puncture through the contact dressing (110) and into the substrate (140).

2. The kit of claim 1, wherein the adhesive is uniformly dispersed on the contact dressing first surface adjacent the inserted insertion needle.

3. The kit of any one of the proceeding claims, wherein the adhesive is discontinuously dispersed on the contact dressing first surface.

4. The kit of any one of the proceeding claims, wherein the antimicrobial agent is uniformly dispersed on the contact dressing first surface adjacent the inserted insertion needle.

5. The kit of any one of the proceeding claims, wherein the antimicrobial agent is discontinuously dispersed on the contact dressing first surface.

6. The kit of any one of the proceeding claims, wherein the antimicrobial agent is dispersed throughout the adhesive.

7. The kit of any one of the proceeding claims, further comprising:
a cover dressing comprising a cover dressing first surface and a cover dressing second surface, opposite the cover dressing first surface, wherein the cover dressing first surface comprises a cover dressing adhesive; and
wherein the cover dressing first surface is adjacent the housing.

8. The kit of claim 7, wherein the cover dressing adhesive at the cover dressing first surface contacts at least a portion of the contact dressing second surface.

9. The kit of any one of the proceeding claims, wherein the insertion needle is configured to protrude from the housing and to puncture through the contact dressing as the access needle hub comes into contact with the contact dressing second surface.

10. The kit of claim 9, wherein the insertion needle is configured to be retracted within the housing and to be actuated to release from within the housing as the access needle hub comes into contact with the contact dressing second surface.

11. The kit of claim 10, wherein the insertion needle is a linear insertion needle configured to linearly project out of the housing to create a punch hole in the contact dressing.

12. The kit of claim 10, wherein the insertion needle is a slightly curved insertion needle configured to linearly project out of the housing to create a linear slit (118) in the contact dressing.

13. The kit claim 10, wherein the insertion needle is configured to rotationally project out of the housing to form a linear slit (118) through the contact dressing.

14. The kit of any one of the proceeding claims, wherein the access needle hub is configured to deliver fluid through the insertion needle and into the substrate/skin and/or removes fluid from the underlying substrate/skin.

15. The kit of any one of the proceeding claims, further comprising:
an access port (150);
wherein the insertion needle is inserted through the contact dressing and into the access port.

## Patentansprüche

1. Ein Kit, aufweisend:
eine Zugangsnadelanordnung (130), aufweisend ein Gehäuse (132) und eine Einführnadel (134); und
eine antimikrobielle Vorrichtung (100), aufweisend:
einen Kontaktwundverband (110), aufweisend:
eine erste Kontaktwundverbandoberfläche (112), aufweisend einen Klebstoff (114) zum Befestigen der antimikrobiellen Vorrichtung an einem Substrat (140) und ein antimikrobielles Mittel (115); und
eine zweite Kontaktwundverbandoberfläche (116) gegenüber der ersten Kontaktwundverbandoberfläche;
wobei das Gehäuse (132) der zweiten Kontaktwundverbandoberfläche (116) angrenzend ist und die Einführnadel (134) durch den Kontaktwundverband (110) eingeführt ist;
wobei sich der Klebstoff (114) und das antimikrobielle Mittel (115) auf der ersten Kontaktwundverbandoberfläche angrenzend an die eingeführte Einführnadel (134) befinden; und
**dadurch gekennzeichnet, dass**
die Einführnadel (134) konfiguriert ist, um durch den Kontaktwundverband (110) hindurch und in das Substrat (140) zu stechen.

2. Das Kit nach Anspruch 1, wobei der Klebstoff auf der ersten Kontaktwundverbandoberfläche angrenzend an die eingeführte Einführnadel gleichmäßig dispergiert ist.

3. Das Kit nach einem der vorstehenden Ansprüche, wobei der Klebstoff diskontinuierlich auf der ersten Kontaktwundverbandoberfläche dispergiert ist.

4. Das Kit nach einem der vorstehenden Ansprüche, wobei das antimikrobielle Mittel auf der ersten Kontaktwundverbandoberfläche angrenzend an die eingeführte Einführnadel gleichmäßig dispergiert ist.

5. Das Kit nach einem der vorstehenden Ansprüche, wobei das antimikrobielle Mittel auf der ersten Kontaktwundverbandoberfläche diskontinuierlich dispergiert ist.

6. Das Kit nach einem der vorstehenden Ansprüche, wobei das antimikrobielle Mittel in dem Klebstoff durchgehend dispergiert ist.

7. Das Kit nach einem der vorstehenden Ansprüche, ferner aufweisend:
einen Abdeckwundverband, aufweisend eine erste Abdeckwundverbandoberfläche und eine zweite Abdeckwundverbandoberfläche gegenüber der ersten Abdeckwundverbandoberfläche, wobei die erste Abdeckwundverbandoberfläche einen Abdeckwundverbandklebstoff aufweist; und
wobei die erste Abdeckwundverbandoberfläche zu dem Gehäuse benachbart ist.

8. Das Kit nach Anspruch 7, wobei Abdeckwundverbandklebstoff an der ersten Abdeckwundverbandoberfläche mindestens einen Abschnitt der zweiten Kontaktwundverbandoberfläche kontaktiert.

9. Das Kit nach einem der vorstehenden Ansprüche, wobei die Einführnadel konfiguriert ist, um aus dem Gehäuse herauszuragen und den Kontaktwundverband zu durchstechen, wenn die Zugangsnadelanordnung mit der zweiten Kontaktwundverbandoberfläche in Kontakt kommt.

10. Das Kit nach Anspruch 9, wobei die Einführnadel konfiguriert ist, um innerhalb des Gehäuses zurückgezogen zu werden und betätigt zu werden, um aus dem Inneren des Gehäuses freigegeben zu werden, wenn die Zugangsnadelanordnung mit der zweiten Kontaktwundverbandoberfläche in Kontakt kommt.

11. Das Kit nach Anspruch 10, wobei die Einführnadel eine lineare Einführnadel ist, die konfiguriert ist, um aus dem Gehäuse linear herauszuragen, um ein Stanzloch in dem Kontaktwundverband zu erzeugen.

12. Das Kit nach Anspruch 10, wobei die Einführnadel eine leicht gekrümmte Einführnadel ist, die konfiguriert ist, um aus dem Gehäuse linear herauszuragen, um einen linearen Schlitz (118) in dem Kontaktwundverband zu erzeugen.

13. Das Kit nach Anspruch 10, wobei die Einführnadel konfiguriert ist, um aus dem Gehäuse rotierend herauszuragen, um einen linearen Schlitz (118) durch den Kontaktwundverband auszubilden.

14. Das Kit nach einem der vorstehenden Ansprüche, wobei die Zugangsnadelanordnung konfiguriert ist, um Fluid durch die Einführnadel und in das Substrat/die Haut abzugeben, und/oder Fluid aus dem darunterliegenden Substrat/der darunterliegenden Haut zu entfernen.

15. Das Kit nach einem der vorstehenden Ansprüche, ferner aufweisend:
einen Zugangsport (150);
wobei die Einführnadel durch den Kontaktwundverband und in den Zugangsport eingeführt wird.

## Revendications

1. Kit, comprenant
une partie centrale à aiguille d'accès (130) comprenant un boîtier (132) et une aiguille d'insertion (134) ; et
un dispositif antimicrobien (100), comprenant :
un pansement de contact (110) comprenant :
une première surface de pansement de contact (112) comprenant un adhésif (114) pour la fixation du dispositif antimicrobien à un substrat (140) et un agent antimicrobien (115) ; et
une seconde surface de pansement de contact (116), opposée à la première surface de pansement de contact ;
dans lequel le boîtier (132) est adjacent à la seconde surface de pansement de contact (116) et l'aiguille d'insertion (134) est insérée à travers le pansement de contact (110) ;
dans lequel l'adhésif (114) et l'agent antimicrobien (115) sont situés sur la première surface de pansement de contact de manière à être adjacents à l'aiguille d'insertion (134) insérée ; et
**caractérisé en ce que**
l'aiguille d'insertion (134) est conçue pour percer à travers le pansement de contact (110) et dans le substrat (140).

2. Kit selon la revendication 1, dans lequel l'adhésif est dispersé uniformément sur la première surface de pansement de contact de manière à être adjacent à l'aiguille d'insertion insérée.

3. Kit selon l'une quelconque des revendications précédentes, dans lequel l'adhésif est dispersé de manière discontinue sur la première surface de pansement de contact.

4. Kit selon l'une quelconque des revendications précédentes, dans lequel l'agent antimicrobien est dispersé uniformément sur la première surface de pansement de contact de manière à être adjacent à l'aiguille d'insertion insérée.

5. Kit selon l'une quelconque des revendications précédentes, dans lequel l'agent antimicrobien est dispersé de manière discontinue sur la première surface de pansement de contact.

6. Kit selon l'une quelconque des revendications précédentes, dans lequel l'agent antimicrobien est dispersé à travers l'adhésif.

7. Kit selon l'une quelconque des revendications précédentes, comprenant en outre :
un pansement de couverture comprenant une première surface de pansement de couverture et une seconde surface de pansement de couverture, opposée à la première surface de pansement de couverture, dans lequel la première surface de pansement de couverture comprend un adhésif de pansement de couverture ; et
dans lequel la première surface de pansement de couverture est adjacente au boîtier.

8. Kit selon la revendication 7, dans lequel l'adhésif de pansement de couverture sur la première surface de pansement de couverture entre en contact avec au moins une partie de la seconde surface de pansement de contact.

9. Kit selon l'une quelconque des revendications précédentes, dans lequel l'aiguille d'insertion est conçue pour faire saillie hors du boîtier et pour percer le pansement de contact lorsque la partie centrale à aiguille d'accès entre en contact avec la seconde surface de pansement de contact.

10. Kit selon la revendication 9, dans lequel l'aiguille d'insertion est conçue pour être rétractée à l'intérieur du boîtier et pour être actionnée pour être libérée de l'intérieur du boîtier lorsque la partie centrale à aiguille d'accès entre en contact avec la seconde surface de pansement de contact.

11. Kit selon la revendication 10, dans lequel l'aiguille d'insertion est une aiguille d'insertion linéaire conçue pour faire saillie linéairement hors du boîtier afin de créer un trou de perforation dans le pansement de contact.

12. Kit selon la revendication 10, dans lequel l'aiguille d'insertion est une aiguille d'insertion légèrement incurvée conçue pour faire saillie linéairement hors du boîtier afin de créer une fente linéaire (118) dans le pansement de contact.

13. Kit selon la revendication 10, dans lequel l'aiguille d'insertion est conçue pour faire saillie en rotation hors du boîtier afin de former une fente linéaire (118) à travers le pansement de contact.

14. Kit selon l'une quelconque des revendications précédentes, dans lequel la partie centrale à aiguille d'accès est conçue pour distribuer un fluide à travers l'aiguille d'insertion et dans le substrat/la peau et/ou retirer du fluide du substrat sous-jacent/de la peau sous-jacente.

15. Kit selon l'une quelconque des revendications précédentes, comprenant en outre :
un orifice d'accès (150) ;
dans lequel l'aiguille d'insertion est insérée à travers le pansement de contact et dans l'orifice d'accès.
